# EUROPÄISCHE PATENTSCHRIFT

(11) **EP 1 951 666 B1**
(45) Veröffentlichungstag und Bekanntmachung des Hinweises auf die Patenterteilung: **28.01.2009**
(21) Anmeldenummer: 06806626.5
(22) Anmeldetag: 31.10.2006
(51) Int. Cl.: C07D 207/28, C09K 8/00

(54) **PYROGLUTAMINSÄUREESTER MIT VERBESSERTER BIOLOGISCHER ABBAUBARKEIT**
PYROGLUTAMIC ACID ESTERS WITH IMPROVED BIODEGRADABILITY
ESTERS D'ACIDE PYROGLUTAMIQUE A BIODEGRADABILITE AMELIOREE

(30) Priorität: 12.11.2005 DE 102005054038
(43) Veröffentlichungstag der Anmeldung: 06.08.2008
(73) Patentinhaber: Clariant Produkte (Deutschland) GmbH, 65929 Frankfurt am Main (DE)
(72) Erfinder: LEINWEBER, Dirk, 65779 Kelkheim (DE); FEUSTEL, Michael, 55278 Köngernheim (DE)
(74) Vertreter: Mikulecky, Klaus
(86) Internationale Anmeldenummer: PCT/EP2006/010454
(87) Internationale Veröffentlichungsnummer: WO 2007/054225

(56) Entgegenhaltungen:
- WO-A-93/25798
- WO-A-94/12761
- US-A- 3 002 978
- FONTAN YANES: "PRODUCTOS DE CONDENSACION DEL ACIDO GLUTAMICO CON GLICOLES" REVISTA DE PLASTICOS MODERNOS, MADRID, ES, Bd. 21, 1970, Seiten 927-932, XP009080494 ISSN: 0034-8708

## Beschreibung

Die vorliegende Erfindung betrifft Pyroglutaminsäureester und ihre Verwendung als Gashydratinhibitoren.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Antiagglomerants). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Antiagglomerants darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

So wird in WO-94/12761 ein Verfahren zur kinetischen Inhibierung der Gashydratbildung durch die Verwendung von Polyvinyllactamen mit einem Molekulargewicht von M_{w} > 40.000 D, und in WO-93/25798 wird ein solches Verfahren unter Verwendung von Polymeren und/oder Copolymeren des Vinylpyrrolidons mit einem Molekulargewicht von M_{w} > 5.000 bis 40.000 D offenbart.

EP-A-0 896 123 offenbart Gashydratinhibitoren, die Copolymere aus alkoxylierter Methacrylsäure ohne Alkyl-Endverschluss und cyclischen N-Vinylverbindungen enthalten können.

In US-5 244 878 wird ein Verfahren zum Verzögern der Bildung oder Vermindern der Bildungstendenz von Gashydraten beschrieben. Hierzu werden Polyole verwendet, die mit Fettsäuren oder Alkenylbernsteinsäureanhydriden verestert werden. Die hergestellten Verbindungen weisen keine Aminosäurefunktionen auf, die mit Clathraten (Käfigmolekülen) interagieren könnten.

Die beschriebenen Additive besitzen nur eingeschränkte Wirksamkeit als kinetische Gashydratinhibitoren und/oder Antiagglomerants, müssen mit Co-Additiven verwendet werden, oder sind nicht in ausreichender Menge oder nur zu hohen Preisen erhältlich.

Um Gashydratinhibitoren auch bei stärkerer Unterkühlung als derzeit möglich, d.h. weiter innerhalb der Hydratregion einsetzen zu können, bedarf es einer weiteren Wirkungssteigerung im Vergleich zu den Hydratinhibitoren des Standes der Technik. Zusätzlich sind in Bezug auf ihre biologische Abbaubarkeit verbesserte Produkte erwünscht.

Aufgabe der vorliegenden Erfindung war es also, Additive zu finden, die sowohl die Bildung von Gashydraten verlangsamen (kinetische Inhibitoren) als auch Gashydratagglomerate klein und pumpbar halten (Antiagglomerants), um so ein breites Anwendungsspektrum mit hohem Wirkpotential zu gewährleisten. Des Weiteren sollten sie in der Lage sein, die derzeit verwendeten thermodynamischen Inhibitoren (Methanol und Glykole), die beträchtliche Sicherheitsprobleme und Logistikprobleme verursachen, zu ersetzen.

Da derzeit verwendete Inhibitoren wie Polyvinylpyrrolidon und Polyvinylcaprolactam nur eine mäßige biologische Abbaubarkeit aufweisen, sollten die erfindungsgemäßen Verbindungen zudem eine verbesserte biologische Abbaubarkeit aufweisen.

Im Stand der Technik sind Pyroglutaminsäureester bekannt, die nicht als Gashydratinhibitoren sondern auf anderen Gebieten Verwendung finden.

So offenbart US-3 565 794 Ester und Amide der Pyroglutaminsäure, welche Amino-, Alkoxy-, Aryloxy-, Cycloalkoxy- oder Heteroalkoxygruppen umfassen, und deren Verwendung als selektives Lösemittel für Kohlenwasserstoffe in der Aromatenextraktion.

US-4 774 255 offenbart Pyroglutaminsäureester von α-Hydroxycarbonsäuren, die ihrerseits mit Mono- oder Polyolen verestert werden. Die erhaltenen Verbindungen enthalten stets mindestens die gleiche Anzahl Esterfunktionen wie sie Pyroglutaminsäure-Struktureinheiten enthalten.

DE-A-25 58 909 offenbart Verbindungen, die durch Umsetzung von Pyroglutaminsäure mit ein- oder mehrwertigen Alkoholen, und nachfolgender Alkoxylierung des erhaltenen Esters entstehen. Diese Verbindungen sind am Stickstoffatom der Pyroglutaminsäure alkoxyliert, und enthalten keine Alkoxygruppen, die die Pyroglutaminsäure-Struktureinheiten an die Alkohol-Struktureinheiten binden.

DE-A-21 02 171 offenbart Pyroglutaminsäureester mit gesättigten C₁₉- bis C₃₀-Alkoholen, und deren therapeutische und kosmetische Anwendung auf der Haut.

JP-A-48-082 046 offenbart Pyroglutaminsäureester von Polyglykolen ohne Endverschluss, sowie Polyolen ohne Alkoxygruppen.

J. Fontán: "Productos de condensación del ácido glutámico con glicoles" Revista de Plásticos Modernos, Bd. 21, 1970, Seiten 927-932 offenbart die Verbindungen 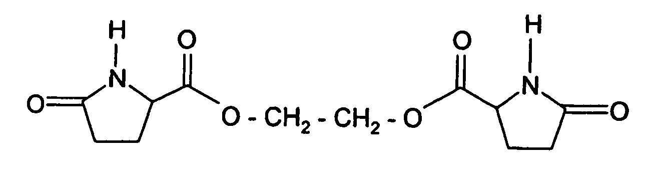 und

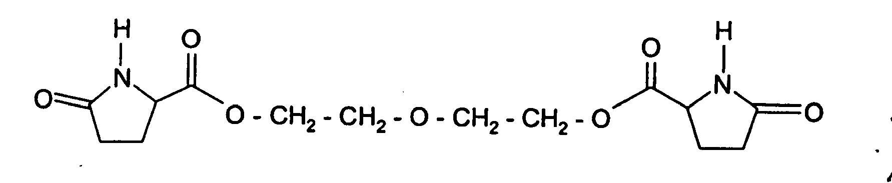

Wie nun überraschenderweise gefunden wurde, eignen sich sowohl wasserlösliche als auch öllösliche Pyroglutaminsäureester als Gashydratinhibitoren. Diese Ester können je nach Struktur sowohl die Keimbildung und das Wachstum von Gashydraten verzögern (kinetische Gashydratinhibitoren) als auch die Agglomeration von Gashydraten unterdrücken (Antiagglomerants). Zudem weisen die erfindungsgemäßen Verbindungen eine deutlich verbesserte biologische Abbaubarkeit auf.

Gegenstand der vorliegenden Erfindung sind Verbindungen der Formel 1 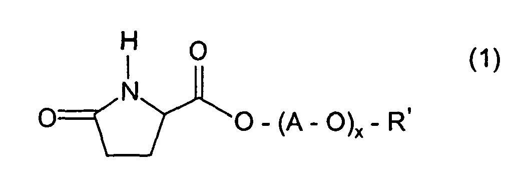 worin
- A: eine C₂- bis C₄-Alkylengruppe
- x: eine Zahl von 1 bis 100
- R': ein aliphatischer, cycloaliphatischer oder aromatischer Rest, welcher mindestens eine Struktureinheit der Formel 2 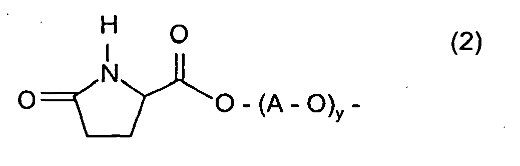 enthält, und
- y: eine Zahl von 0 bis 100 bedeuten.
mit der Maßgabe, dass Verbindungen ausgeschlossen sind, in denen R' für die Struktureinheit der Formel 2, x für eins und y für null oder eins stehen.

Ein weiterer Gegenstand der Erfindung ist die Verwendung von Verbindungen der Formel 1 in Mengen von 0,01 bis 2 Gew.-% (bezogen auf das Gewicht der wässrigen Phase) zur Verhinderung der Bildung von Gashydraten in wässrigen Phasen, die mit einer gasförmigen, flüssigen oder festen organischen Phase in Verbindung stehen.

Ein weiterer Gegenstand der Erfindung ist ein Verfahren zur Inhibierung der Bildung von Gashydraten, indem man einer mit einer gasförmigen, flüssigen oder festen organischen Phase in Verbindung stehenden wässrigen Phase, in der die Gashydratbildung verhindert werden soll mindestens eine Verbindung der Formel 1 in Mengen von 0,01 bis 2 Gew.-% (bezogen auf das Gewicht der wässrigen Phase) zusetzt.

R' steht für einen organischen Rest, der eine oder mehrere Struktureinheiten der Formel 2 enthält. Er kann im Allgemeinen durch die Formel 3 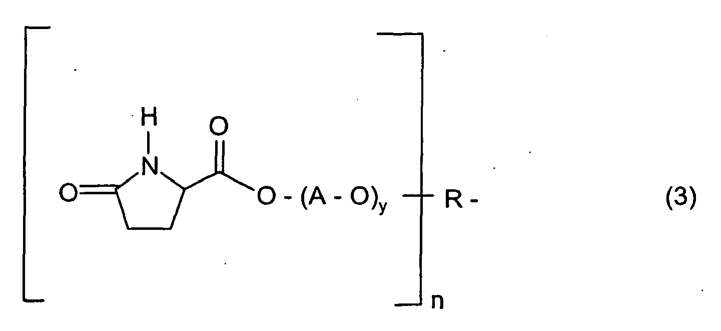 wiedergegeben werden, worin n für 1 - 100 steht, und R ein aliphatischer, cycloaliphatischer oder aromatischer Rest ist, welcher Heteroatome enthalten kann und mindestens (n+1) aktive Wasserstoffatome trägt, die durch Alkoxylierung substituiert werden können, und der n Struktureinheiten der Formel 2 trägt. R steht bevorzugt für einen organischen Rest mit zwei bis sechs aktiven Wasserstoffatomen. Die aktiven Wasserstoffatome können beispielsweise aus Hydroxylgruppen, Aminogruppen oder Carboxylgruppen stammen, welche an Alkyl-, Alkenyl-, Cycloalkyl-, Aryl-, Alkylaryl- oder Polyoxyalkylenreste gebunden sind.

R steht in einer bevorzugten Ausführungsform für einen organischen Rest mit zwei aktiven Wasserstoffatomen und bis zu 100 Kohlenstoffatomen. Demzufolge beträgt n dann 1.

In einer bevorzugten Ausführungsform der Erfindung ist R ein Rest, der höchstens eine Estergruppe oder höchstens eine Carbonsäuregruppe umfasst. Damit ist gemeint, dass R entweder eine Estergruppe oder eine Carbonsäuregruppe oder weder eine Estergruppe noch eine Carbonsäuregruppe umfasst. R kann beliebige weitere Substituenten umfassen, beispielsweise Amino- oder Amidosubstituenten. R steht besonders bevorzugt für einen Alkylen- oder Polyalkylenoxid-Rest mit 1 bis 30 Kohlenstoffatomen.

In einer weiteren bevorzugten Ausführungsform ist R ein durch formale Abstraktion der Wasserstoffatome der OH-Gruppen eines Polyols entstehender Rest. Bevorzugte Polyole sind Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Propylenglykol, Dipropylenglykol und Tripropylenglykol. Es können alle oder nur ein Teil der Wasserstoffatome der Polyole bei der Bildung von R abstrahiert werden. Bevorzugt ist es, wenn 2, 3, 4, 5, oder 6 Wasserstoffatome der OH-Gruppen des Polyols abstrahiert werden, d.h. Reste der Formel 2 tragen.

R kann auch eine Einfachbindung sein. In diesem Fall wird R' durch die Formel 2 wiedergegeben.

A steht vorzugsweise für Ethylenreste oder für Mischungen aus Ethylen- und Propylenresten.

In der durch (A-O)ₓ oder (A-O)_{y} wiedergegebenen Alkoxykette bedeutet A vorzugsweise einen Ethylen- oder Propylenrest, insbesondere einen Ethylenrest. x und y bedeuten vorzugsweise unabhängig voneinander eine Zahl zwischen 1 und 80, insbesondere zwischen 2 und 70, speziell zwischen 3 und 50. Bei der Alkoxykette kann es sich um eine Blockpolymerkette handeln, die alternierende Blöcke verschiedener Alkoxyeinheiten, vorzugsweise Ethoxy- und Propoxyeinheiten, aufweist. Es kann sich dabei auch um eine Kette mit statistischer Abfolge der Alkoxyeinheiten oder ein Homopolymer handeln.

In einer bevorzugten Ausführungsform steht -(A-O)ₓ- oder -(A-O)_{y}- für eine Alkoxykette der Formel

―(CH₂ - CH₂ - O)ₐ -- (CH₂ - CH(CH₃) - O)_{b} -- (CH₂ - CH₂ - O)_{c}―

worin
- a: eine Zahl von 1 bis 100, vorzugsweise 5 bis 80
- b: eine Zahl von 0 bis 100, vorzugsweise 5 bis 100
- c: eine Zahl von 1 bis 100, vorzugsweise 5 bis 80 bedeuten.

In einer weiteren bevorzugten Ausführungsform steht -(A-O)ₙ- für einen Ethoxyrest mit 1 bis 100 Ethoxyeinheiten.

Allen Ausführungsformen gemein ist, dass vorzugsweise mindestens 50 mol-% der Reste (A-O) Ethoxyreste sind, insbesondere sind 60 bis 100 mol-% Ethoxyreste.

Beispiele erfindungsgemäßer Verbindungen sind in den folgenden Formeln 4 und 5 angegeben: 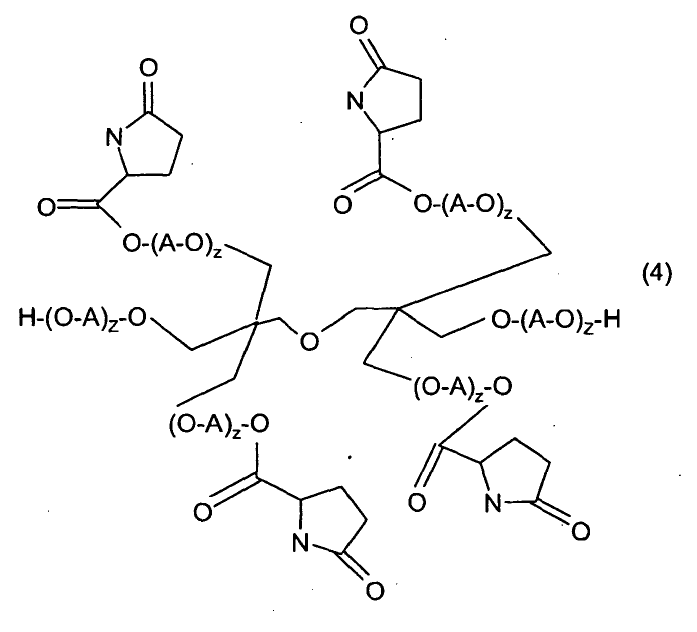 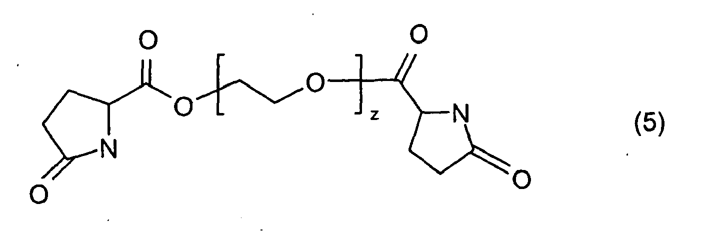 worin z die für x und y angegebenen Werte annehmen kann. Formel 5 entspricht dem Fall, wenn R eine Einfachbindung ist.

Die erfindungsgemäßen Pyroglutaminsäureester sind durch Veresterung von Pyroglutaminsäure (enantiomerenrein oder racemisch) oder Glutaminsäure (vorzugsweise L-Glutaminsäure) mit mindestens einem Alkohol der Formel HO-(A-O)ₓ-R-(O-A)_{y}-OH darstellbar. Die Herstellung der erfindungsgemäßen Ester ist im Stand der Technik bekannt und erfolgt durch unkatalysierte oder sauer katalysierte Kondensation der Carbonsäure mit dem entsprechenden Alkohol. Die Reaktionstemperatur liegt im Allgemeinen zwischen 100 und 300°C, vorzugsweise bei 170 bis 250°C.

Das bei der Veresterung zur Anwendung kommende molare Verhältnis von OH-Gruppen im Alkohol zur Pyroglutaminsäure oder Glutaminsäure liegt vorzugsweise zwischen 1 : 0,3 und 1:1, insbesondere zwischen 1 : 0,5 und 1:1.

Die Reaktion kann bei Atmosphärendruck oder vermindertem Druck durchgeführt werden. Als katalysierende Säuren sind beispielsweise HCl, H₂SO₄, Sulfonsäuren, H₃PO₄ oder saure Ionentauscher zu nennen, die in Mengen von 0,1 bis 5 Gew.-%, bezogen auf das Gewicht des Reaktionsgemisches, verwendet werden. Die Veresterung nimmt im Allgemeinen 3 bis 30 Stunden in Anspruch.

Zur Herstellung der erfindungsgemäßen Pyroglutaminsäureester kann neben Pyroglutaminsäure auch Glutaminsäure verwendet werden, welche direkt zum Pyroglutaminsäureester umgesetzt wird. Dementsprechend werden dann 2 mol Reaktionswasser pro veresterter Alkoholfunktion gebildet. Als Nebenprodukte können in diesem Fall auch Glutaminsäureester gebildet werden, die die erfindungsgemäße Verwendung der Pyroglutaminsäureester als Gashydratinhibitoren aber nicht stören.

Die erfindungsgemäßen Pyroglutaminsäureester können alleine oder in Kombination mit anderen bekannten Gashydratinhibitoren eingesetzt werden. Im Allgemeinen wird man dem zur Hydratbildung neigenden System soviel des erfindungsgemäßen Pyroglutaminsäureesters zusetzen, dass man unter den gegebenen Druck- und Temperaturbedingungen eine ausreichende Inhibierung erhält. Die erfindungsgemäßen Pyroglutaminsäureester werden vorzugsweise in Mengen zwischen 0,02 bis 1 Gew.-% (bezogen auf das Gewicht der wässrigen Phase) verwendet. Werden die erfindungsgemäßen Pyroglutaminsäureester in Mischung mit anderen Gashydratinhibitoren verwendet, so beträgt die Konzentration der Mischung 0,01 bis 2 bzw. 0,02 bis 1 Gew.-% in der wässrigen Phase.

Die Pyroglutaminsäureester werden vorzugsweise zur Anwendung als Gashydratinhibitoren in wassermischbaren alkoholischen Lösungsmitteln wie beispielsweise Methanol, Ethanol, Propanol, Butanol, Ethylenglykol sowie oxethylierten Monoalkoholen, wie Butylglykol, Isobutylglykol, Butyldiglykol gelöst.

### Beispiele

### Herstellung der Pyroglutaminsäureester

### Beispiel 1

### Herstellung von Triethylenglykol-di(pyroglutamat)

In einem 250 ml Vierhalskolben mit Rührer, Thermometer, Stickstoffspülung sowie Destillationsbrücke wurden 129 g Pyroglutaminsäure, 75 g Triethylenglykol sowie 1,0 g p-Toluolsulfonsäure vermischt und auf 200°C erhitzt. Innerhalb von 8 h bei 200°C wurden ca. 18 ml Wasser abdestilliert. Dabei wurden ca. 187 g Triethylenglykol-di(pyroglutamat) mit einer Verseifungszahl von 303 mg KOH/g erhalten.

### Beispiel 2

### Herstellung von Triethylenglykol-di(pyroglutamat) - Variante mit L-Glutaminsäure

In einem 250 ml Vierhalskolben mit Rührer, Thermometer, Stickstoffspülung sowie Destillationsbrücke wurden 147 g L-Glutaminsäure, 75 g Triethylenglykol sowie 1,0 g p-Toluolsulfonsäure vermischt und auf 200°C erhitzt. Innerhalb von 12 h bei 200°C wurden ca. 36 ml Wasser abdestilliert. Dabei wurden ca. 187 g Triethylenglykol-di(pyroglutamat) mit einer Verseifungszahl von 303 mg KOH/g erhalten.

### Beispiel 3

### Herstellung von (Trimethylolpropan + 9 EO)-tri(pyroglutamat)

In einem 500 ml Vierhalskolben mit Rührer, Thermometer, Stickstoffspülung sowie Destillationsbrücke wurden 129 g Pyroglutaminsäure, 265 g Trimethylolpropan + 9 EO sowie 1,5 g p-Toluolsulfonsäure vermischt und auf 200°C erhitzt. Innerhalb von 15 h bei 200°C wurden ca. 18 ml Wasser abdestilliert. Dabei wurden ca. 376 g (Trimethylolpropan + 9 EO)-tri(pyroglutamat) mit einer Verseifungszahl von 148 mg KOH/g erhalten.

### Beispiel 4

### Herstellung von (Sorbitol + 18 EO)-tri(pyroglutamat)

In einem 1000 ml Vierhalskolben mit Rührer, Thermometer, Stickstoffspülung sowie Destillationsbrücke wurden 129 g Pyroglutaminsäure, 325 g Sorbitol + 18 EO sowie 1,5 g p-Toluolsulfonsäure vermischt und auf 200°C erhitzt. Innerhalb von 20 h bei 200°C wurden ca. 18 ml Wasser abdestilliert. Dabei wurden ca. 436 g (Sorbitol + 18 EO)-tri(pyroglutamat) mit einer Verseifungszahl von 127 mg KOH/g erhalten.

### Beispiel 5

### Herstellung von (Sorbitol + 30 EO)-hexa(pyroglutamat)

In einem 500 ml Vierhalskolben mit Rührer, Thermometer, Stickstoffspülung sowie Destillationsbrücke wurden 129 g Pyroglutaminsäure, 250 g Sorbitol + 30 EO sowie 1,0 g p-Toluolsulfonsäure vermischt und auf 200°C erhitzt. Innerhalb von 20 h bei 200°C wurden ca. 18 ml Wasser abdestilliert. Dabei wurden ca. 361 g (Sorbitol + 30 EO)-hexa(pyroglutamat) mit einer Verseifungszahl von 154 mg KOH/g erhalten.

### Beispiel 6

### Herstellung von (Dekaglycerin + 30 EO)-poly(pyroglutamat)

In einem 1000 ml Vierhalskolben mit Rührer, Thermometer, Stickstoffspülung sowie Destillationsbrücke wurden 129 g Pyroglutaminsäure, 390 g Dekaglycerin + 30 EO sowie 1,5 g p-Toluolsulfonsäure vermischt und auf 200°C erhitzt. Innerhalb von 30 h bei 200°C wurden ca. 18 ml Wasser abdestilliert. Dabei wurden ca. 501 g (Dekaglycerin + 30 EO)-poly(pyroglutamat) mit einer Verseifungszahl von 112 mg KOH/g erhalten.

### Beispiel 7

### Herstellung von (Polypropylenglykol 400 + 10 EO)-di(pyroglutamat)

In einem 1000 ml Vierhalskolben mit Rührer, Thermometer, Stickstoffspülung sowie Destillationsbrücke wurden 129 g Pyroglutaminsäure, 420 g Polypropylenglykol 400 + 10 EO sowie 1,5 g p-Toluolsulfonsäure vermischt und auf 200°C erhitzt. Innerhalb von 18 h bei 200°C wurden ca. 18 ml Wasser abdestilliert. Dabei wurden ca. 531 g (Polypropylenglykol 400 + 10 EO)-di(pyroglutamat) mit einer Verseifungszahl von 105 mg KOH/g erhalten.

### Beispiel 8

### Herstellung von (Polypropylenglykol 600)-di(pyroglutamat)

In einem 500 ml Vierhalskolben mit Rührer, Thermometer, Stickstoffspülung sowie Destillationsbrücke wurden 129 g Pyroglutaminsäure, 300 g Polypropylenglykol 600 sowie 1,0 g p-Toluolsulfonsäure vermischt und auf 200°C erhitzt. Innerhalb von 20 h bei 200°C wurden ca. 18 ml Wasser abdestilliert. Dabei wurden ca. 411 g (Polypropylen-glykol 600)-di(pyroglutamat) mit einer Verseifungszahl von 136 mg KOH/g erhalten.

### Wirksamkeit der Polymere als Gashydratinhibitoren

Zur Untersuchung der inhibierenden Wirkung der Polyester wurde ein Stahlrührautoklav mit Temperatursteuerung, Druck- und Drehmomentaufnehmer mit 450 ml Innenvolumen benutzt. Für Untersuchungen zur kinetischen Inhibierung wurde der Autoklav mit destillierten Wasser und Gas im Volumenverhältnis 20:80 gefüllt, für Untersuchungen der Agglomeratinhibierung wurde zusätzlich Kondensat zugegeben. Anschließend wurden 50 bar Erdgas aufgedrückt.

Ausgehend von einer Anfangstemperatur von 20°C wurde innerhalb 3 h auf 4°C abgekühlt, dann 18 h bei 4°C gerührt und innerhalb von 2 h wieder auf 20°C aufgeheizt. Dabei wird zunächst eine Druckabnahme gemäß der thermischen Kompression des Gases beobachtet. Tritt während der Unterkühlzeit die Bildung von Gashydratkeimen auf, so verringert sich der gemessene Druck, wobei ein Anstieg des gemessenen Drehmoments und ein leichter Anstieg der Temperatur zu beobachten ist. Weiteres Wachstum und zunehmende Agglomeration der Hydratkeime führt ohne Inhibitor schnell zu einem weiteren Anstieg des Drehmomentes. Beim Aufwärmen des Gemisches zerfallen die Gashydrate, so dass man den Anfangszustand der Versuchsreihe erreicht.
Als Maß für die inhibierende Wirkung des Polymers wird die Zeit vom Erreichen der Minimaltemperatur von 4°C bis zur ersten Gasaufnahme (T_{ind}) bzw. die Zeit bis zum Anstieg des Drehmomentes (T_{agg}) benutzt. Lange Induktionszeiten bzw. Agglomerationszeiten weisen auf eine Wirkung als kinetischer Inhibitor hin. Das im Autoklaven gemessene Drehmoment dient dagegen als Größe für die Agglomeration der Hydratkristalle. Bei einem guten Antiagglomerant ist das Drehmoment, das sich nach Bildung von Gashydraten aufbaut, gegenüber dem Blindwert deutlich verringert. Im Idealfall bilden sich schneeartige, fein-verteilte Hydratkristalle in der Kondensatphase, die sich nicht zusammenlagern und somit nicht zum Verstopfen der zum Gastransport und zur Gasförderung dienenden Installationen führen.

### Testergebnisse

### Zusammensetzung des verwendeten Erdgases:

Methan 84,8 %, Ethan 9,2 %, Propan 2,6 %, Butan 0,9 %, Kohlendioxid 1,6 %, Stickstoff 0,9 %.

Als Vergleichssubstanz wurde ein kommerziell erhältlicher Gashydratinhibitor auf Basis Polyvinylpyrrolidon verwendet. Die Dosierrate betrug bei allen Versuchen 5000 ppm bezogen auf die Wasserphase.

| Pyroglutaminsäureester aus Beispiel | T_{ind} (h) | T_{agg} (h) |
|---|---|---|
| Blindwert | 0 | 0 |
| 1 | 6,9 | 7,2 |
| 2 | 7,3 | 7,4 |
| 3 | 8,9 | 9,0 |
| 4 | 8,5 | 8,8 |
| 5 | 7,4 | 7,7 |
| 6 | 9,0 | 9,1 |
| 7 | 5,7 | 6,0 |
| Vergleich | 3,5 | 3,6 |

Wie aus den obigen Testresultaten zu erkennen ist, wirken die erfindungsgemäßen Pyroglutaminsäureester als kinetische Gashydratinhibitoren und zeigen eine deutliche Verbesserung gegenüber dem Stand der Technik.

Um die Wirkung als Agglomeratinhibitoren zu prüfen, wurde im oben verwendeten Testautoklaven Wasser und Testbenzin vorgelegt (20 % des Volumens im Verhältnis 1:2) und bezogen auf die Wasserphase 5000 ppm des jeweiligen Additivs zugegeben.
Bei einem Autoklavendruck von 50 bar und einer Rührgeschwindigkeit von 500 U/min wurde die Temperatur von anfangs 20°C innerhalb 3 Stunden auf 4°C abgekühlt, dann 18 Stunden bei 2°C gerührt und wieder aufgewärmt. Dabei wurden die Agglomerationszeit bis zum Auftreten von Gashydrat-Agglomeraten und das dabei auftretende Drehmoment am Rührer gemessen, das ein Maß für die Agglomeration der Gashydrate ist.

Als Vergleichssubstanz wurde ein kommerziell erhältlicher Anti-Agglomerant (quartäres Ammoniumsalz) herangezogen.

| Pyroglutaminsäureester aus Beispiel | T_{agg} (h) | Mₘₐₓ(Ncm) |
|---|---|---|
| Blindwert | 0,1 | 15,9 |
| 7 | 4,3 | 1,9 |
| 8 | 3,5 | 1,1 |
| Vergleich | 2,6 | 4,1 |

Wie aus diesen Beispielen zu ersehen ist, sind die gemessenen Drehmomente im Vergleich zum Blindwert trotz Gashydratbildung stark reduziert. Dies spricht für eine deutliche agglomerat-inhibierende Wirkung der erfindungsgemäßen Produkte. Zusätzlich weisen die Produkte unter den Versuchsbedingungen auch eine deutliche Wirkung als kinetische Inhibitoren auf. Sämtliche Beispiele zeigen eine deutlich bessere Performance als der kommerziell erhältliche Anti-Agglomerant (Vergleich = Stand der Technik).

Im Folgenden wird die deutlich verbesserte biologische Abbaubarkeit (nach OECD 306) der erfindungsgemäßen Verbindungen im Vergleich zum Stand der Technik (kommerziell erhältliches Polyvinylpyrrolidon) aufgezeigt.

| Pyroglutaminsäureester aus Beispiel | Biologische Abbaubarkeit 28 Tage (OECD 306) |
|---|---|
| Polyvinylpyrrolidon | 5 |
| 1 | 72 |
| 2 | 70 |
| 3 | 65 |
| 4 | 78 |
| 5 | 58 |
| 6 | 42 |
| 7 | 47 |
| 8 | 25 |

## Patentansprüche

1. Verbindungen der Formel 1 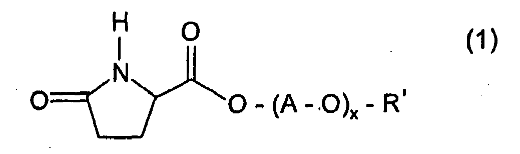 worin
A eine C₂- bis C₄-Alkylengruppe
x eine Zahl von 1 bis 100
R' ein aliphatischer, cycloaliphatischer oder aromatischer Rest, welcher mindestens eine Struktureinheit der Formel 2 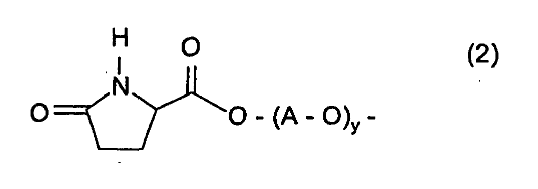 enthält, und
y eine Zahl von 0 bis 100 bedeuten
mit der Maßgabe, dass Verbindungen ausgeschlossen sind, in denen R' für die Struktureinheit der Formel 2, x für eins und y für null oder eins stehen.

2. Verbindungen gemäß Anspruch 1, worin R' von Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Propylenglykol, Dipropylenglykol und Tripropylenglykol abgeleitet ist.

3. Verbindungen gemäß der Ansprüche 1 und/oder 2, wobei zur Herstellung der Pyroglutaminsäureester L-Glutaminsäure in enantiomerenreiner Form verwendet wird.

4. Verbindungen gemäß einem oder mehreren der Ansprüche 1 bis 3, wobei der Pyroglutaminsäureester Glutaminsäureester enthält.

5. Verbindungen gemäß einem oder mehreren der Ansprüche 1 bis 4, wobei x und y unabhängig voneinander eine Zahl von 2 bis 80 bedeuten.

6. Verbindungen gemäß einem oder mehreren der Ansprüche 1 bis 5, wobei R' für einen organischen Rest der Formel 3 steht 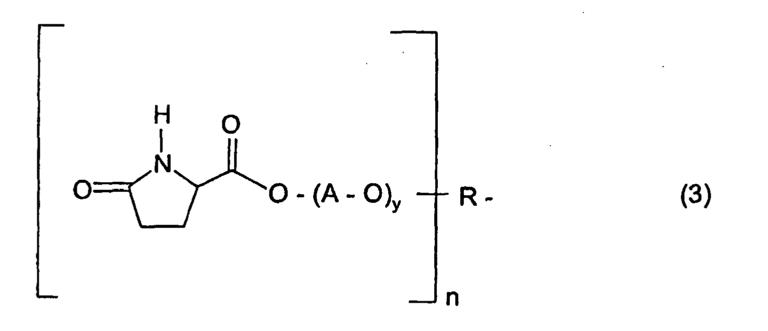 worin n für 1 - 100 steht, und R ein aliphatischer, cycloaliphatischer oder aromatischer Rest ist, welcher Heteroatome enthalten kann und mindestens (n+1) aktive Wasserstoffatome trägt, die durch Alkoxylierung substituiert werden können.

7. Verbindungen gemäß einem oder mehreren der Ansprüche 1 bis 6, wobei A für Ethylenreste, Propylenreste oder Mischungen aus Ethylen- und Propylenresten steht.

8. Verwendung einer Verbindung der Formel 1 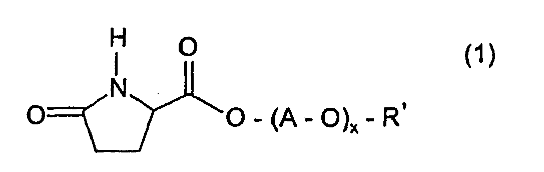 worin
A eine C₂- bis C₄-Alkylengruppe
x eine Zahl von 1 bis 100
R' ein aliphatischer, cycloaliphatischer oder aromatischer Rest, welcher mindestens eine Struktureinheit der Formel 2 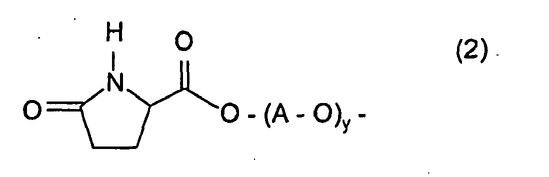 enthält, und
y eine Zahl von 0 bis 100 bedeuten, als Gashydratinhibitor.

9. Verwendung gemäß Anspruch 8, worin R' von Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Propylenglykol, Dipropylenglykol und Tripropylenglykol abgeleitet ist.

10. Verwendung gemäß der Ansprüche 8 und/oder 9, wobei zur Herstellung der Pyroglutaminsäureester L-Glutaminsäure in enantiomerenreiner Form verwendet wird.

11. Verwendung gemäß einem oder mehreren der Ansprüche 8 bis 10, wobei der Pyroglutaminsäureester Glutaminsäureester enthält.

12. Verwendung gemäß einem oder mehreren der Ansprüche 8 bis 11, wobei x und y unabhängig voneinander eine Zahl von 2 bis 80 bedeuten.

13. Verwendung gemäß einem oder mehreren der Ansprüche 8 bis 12, wobei R' für einen organischen Rest der Formel 3 steht 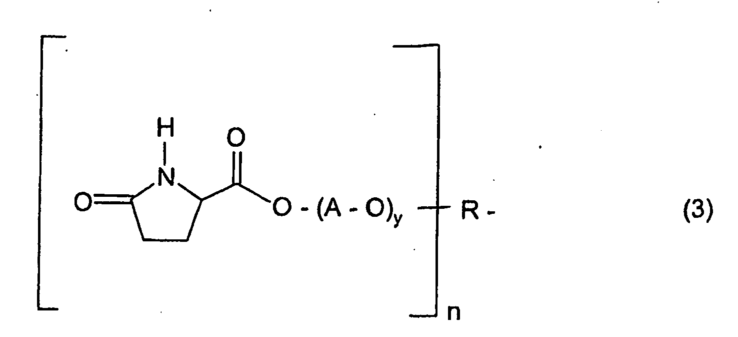 worin n für 1 - 100 steht, und R ein aliphatischer, cycloaliphatischer oder aromatischer Rest ist, welcher Heteroatome enthalten kann und mindestens (n+1) aktive Wasserstoffatome trägt, die durch Alkoxylierung substituiert werden können.

14. Verwendung gemäß einem oder mehreren der Ansprüche 8 bis 13, wobei A für Ethylenreste, Propylenreste oder Mischungen aus Ethylen- und Propylenresten steht.

## Claims

1. A compound of the formula 1 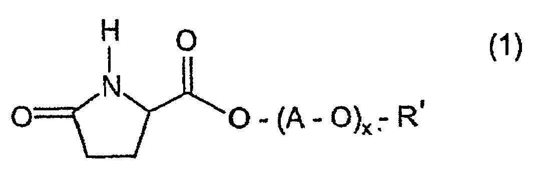 in which
A is a C₂- to C₄-alkylene group
x is from 1 to 100
R' is an aliphatic, cycloaliphatic or aromatic radical which contains at least one structural unit of the formula 2 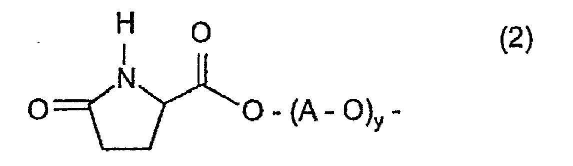 and
y is from 0 to 100,
with the proviso that compounds in which R' is the structural unit of the formula 2, x is one and y is zero or one are excluded.

2. A compound as claimed in claim 1, in which R' is derived from ethylene glycol, propylene glycol, glycerol, diglycerol, triglycerol, tetraglycerol, polyglycerol, trimethylolpropane, pentaerythritol, dipentaerythritol, tripentaerythritol, sorbitol, sorbitan, diethylene glycol, triethylene glycol, propylene glycol, dipropylene glycol and tripropylene glycol.

3. A compound as claimed in claim 1 and/or 2, wherein the pyroglutamic esters are prepared by using L-glutamic acid in enantiomerically pure form.

4. A compound as claimed in one or more of claims 1 to 3, wherein the pyroglutamic ester comprises glutamic esters.

5. A compound as claimed in one or more of claims 1 to 4, wherein x and y are each independently from 2 to 80.

6. A compound as claimed in one or more of claims 1 to 5, wherein R' is an organic radical of the formula 3 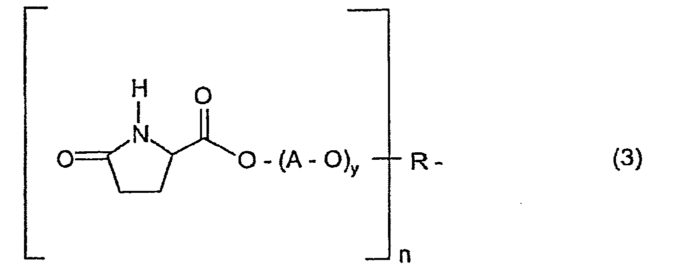 in which n is 1 - 100 and R is an aliphatic, cycloaliphatic or aromatic radical which may contain heteroatoms and bears at least (n+1) active hydrogen atoms which can be substituted by alkoxylation.

7. A compound as claimed in one or more of claims 1 to 6, wherein A represents ethylene radicals, propylene radicals or mixtures of ethylene and propylene radicals.

8. The use of a compound of the formula 1 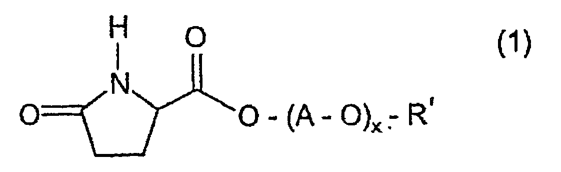 in which
A is a C₂- to C₄-alkylene group
x is from 1 to 100
R' is an aliphatic, cycloaliphatic or aromatic radical which contains at least one structural unit of the formula 2 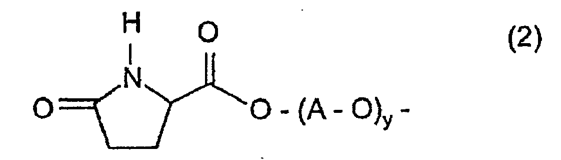 and
y is from 0 to 100 as a gas hydrate inhibitor.

9. The use as claimed in claim 8, in which R' is derived from ethylene glycol, propylene glycol, glycerol, diglycerol, triglycerol, tetraglycerol, polyglycerol, trimethylolpropane, pentaerythritol, dipentaerythritol, tripentaerythritol, sorbitol, sorbitan, diethylene glycol, triethylene glycol, propylene glycol, dipropylene glycol and tripropylene glycol.

10. The use as claimed in claim 8 and/or 9, wherein the pyroglutamic esters are prepared by using L-glutamic acid in enantiomerically pure form.

11. The use as claimed in one or more of claims 8 to 10, wherein the pyroglutamic ester comprises glutamic esters.

12. The use as claimed in one or more of claims 8 to 11, wherein x and y are each independently from 2 to 80.

13. The use as claimed in one or more of claims 8 to 12, wherein R' is an organic radical of the formula 3 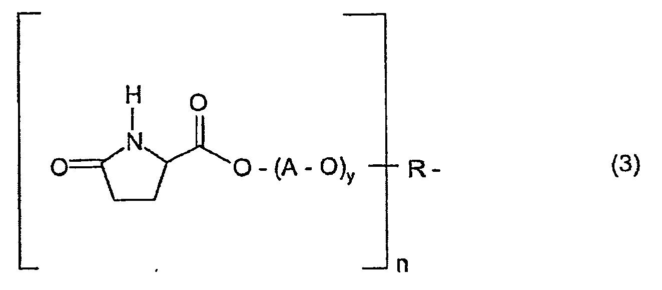 in which n is 1 - 100 and R is an aliphatic, cycloaliphatic or aromatic radical which may contain heteroatoms and bears at least (n+1) active hydrogen atoms which can be substituted by alkoxylation.

14. The use as claimed in one or more of claims 8 to 13, wherein A represents ethylene radicals, propylene radicals or mixtures of ethylene and propylene radicals.

## Revendications

1. Composés de formule 1 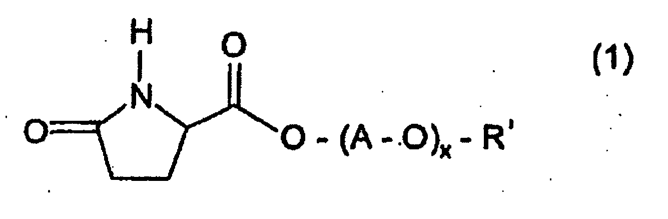 dans laquelle
A représente un groupe alkylène en C₂-C₄
x représente un nombre allant de 1 à 100
R' représente un radical aliphatique, cycloaliphatique ou aromatique qui contient au moins un motif structural de formule 2 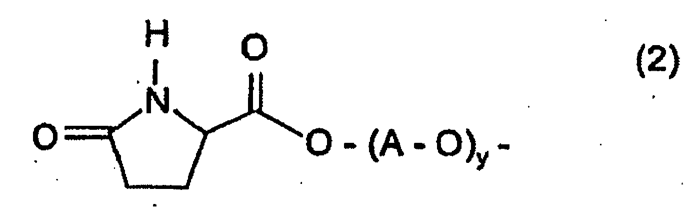 et
y représente un nombre allant de 0 à 100,
étant entendu que sont exclus les composés dans lesquels R' représente le motif structural de formule 2, x représente un et y représente zéro ou un.

2. Composés selon la revendication 1, dans lesquels R' est dérivé d'éthylèneglycol, de propylèneglycol, glycérol, diglycérol, triglycérol, tétraglycérol, polyglycérol, triméthylolpropane, pentaérythritol, dipentaérythritol, tripentaérythritol, sorbitol, sorbitanne, diéthylèneglycol, triéthylèneglycol, propylèneglycol, dipropylèneglycol et tripropylèneglycol.

3. Composés selon la revendication 1 et/ou la revendication 2, dans lesquels pour la préparation des esters d'acide pyroglutamique on utilise de l'acide L-glutamique sous forme d'énantiomère pur.

4. Composés selon une ou plusieurs des revendications 1 à 3, dans lesquels l'ester d'acide pyroglutamique contient de l'ester d'acide glutamique.

5. Composés selon une ou plusieurs des revendications 1 à 4, dans lesquels x et y représentent, indépendamment l'un de l'autre, un nombre allant de 2 à 80.

6. Composés selon une ou plusieurs des revendications 1 à 5, dans lesquels R' représente un radical organique de formule 3 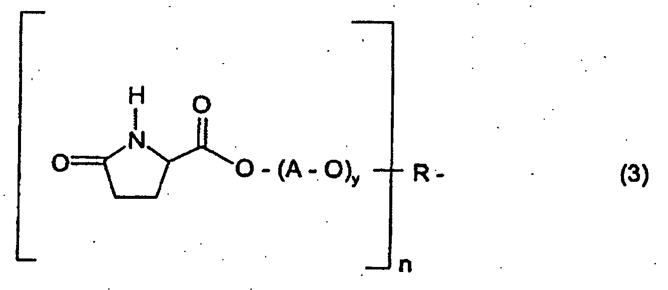 dans laquelle n va de 1 à 100, et R est un radical aliphatique, cycloaliphatique ou aromatique qui peut contenir des hétéroatomes et porte au moins (n+1) atomes d'hydrogène actifs, qui peuvent être remplacés par alcoxylation.

7. Composés selon une ou plusieurs des revendications 1 à 6, dans lesquels A représente des radicaux éthylène, des radicaux propylène ou des mélanges de radicaux éthylène et propylène.

8. Utilisation d'un composé de formule 1 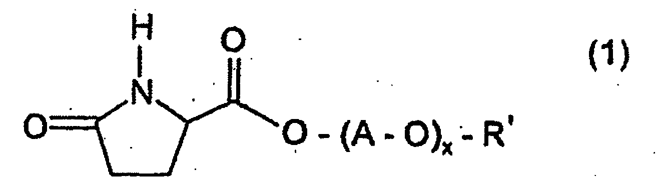 dans laquelle
A représente un groupe alkylène en C₂-C₄
x représente un nombre allant de 1 à 100
R' représente un radical aliphatique, cycloaliphatique ou aromatique qui contient au moins un motif structural de formule 2 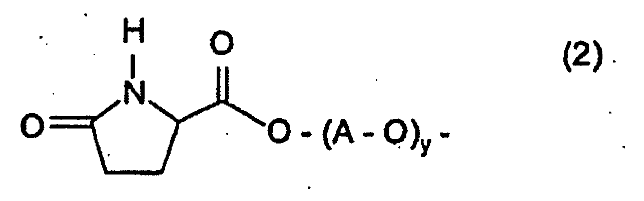 et
y représente un nombre allant de 0 à 100
en tant qu'inhibiteur de la formation d'hydrates de gaz.

9. Utilisation selon la revendication 8, dans laquelle R' est dérivé d'éthylèneglycol, de propylèneglycol, glycérol, diglycérol, triglycérol, tétraglycérol, polyglycérol, triméthylolpropane, pentaérythritol, dipentaérythritol, tripentaérythritol, sorbitol, sorbitanne, diéthylèneglycol, triéthylèneglycol, propylèneglycol, dipropylèneglycol et tripropylèneglycol.

10. Utilisation selon la revendication 8 et/ou la revendication 9, dans laquelle pour la préparation des esters d'acide pyroglutamique on utilise de l'acide L-glutamique sous forme d'énantiomère pur.

11. Utilisation selon une ou plusieurs des revendications 8 à 10, dans laquelle l'ester d'acide pyroglutamique contient de l'acide glutamique.

12. utilisation selon une ou plusieurs des revendications 8 à 11, dans laquelle x et y représentent, indépendamment l'un de l'autre, un nombre allant de 2 à 80.

13. Utilisation selon une ou plusieurs des revendications 8 à 12, dans laquelle R' représente un radical organique de formule 3 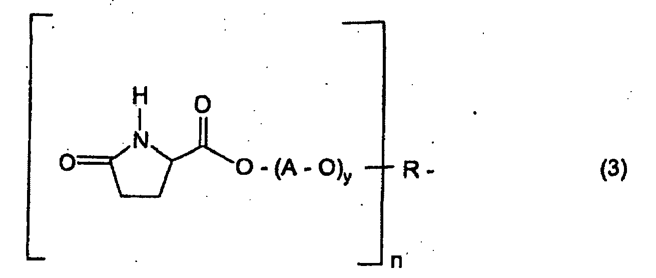 dans laquelle n va de 1 à 100, et R est un radical aliphatique, cycloaliphatique ou aromatique qui peut contenir des hétéroatomes et porte au moins (n+1) atomes d'hydrogène actifs, qui peuvent être remplacés par alcoxylation.

14. Composés selon une ou plusieurs des revendications 8 à 13, dans laquelle A représente des radicaux éthylène, des radicaux propylène ou des mélanges de radicaux éthylène et propylène.
